Europäisches Patentamt

European Patent Office

Office européen des brevets

(19)

(11) **EP 1 271 141 A1**

(12) **EUROPEAN PATENT APPLICATION**

(43) Date of publication:
**02.01.2003 Bulletin 2003/01**

(51) Int Cl.[7]: **G01N 33/18**, C12M 1/00,
C02F 3/00, C02F 3/02

(21) Application number: **01115125.5**

(22) Date of filing: **21.06.2001**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **CHAOYANG UNIVERSITY OF TECHNOLOGY**
**Taichung County, Taiwan (TW)**

(72) Inventors:
• **Chiang, Chow-Feng**
  **Taichung City, (TW)**
• **Wu, Yeong-Shing**
  **Nantour County (TW)**

(74) Representative: **Viering, Jentschura & Partner**
**Postfach 22 14 43**
**80504 München (DE)**

(54) **Apparatus and method for determining biological heat potential of a waste water treatment system.**

(57)      A device and method to determine the biological heat potential consists of an acclimation apparatus for aerobic seed source, a reactor to carry out the desired biological reaction, an external temperature controller to control and heat the ambient air surrounding the reactor at a preset temperature, an oxygen controller to supply and record the oxygen depleted within the reactor with an output of oxygen uptake data, an internal heat controller to control and heat the reactor at a preset temperature with an output of heat compensation data. Based on the oxygen uptake data and heat compensation data, uses a specific biological heat potential evaluator to compute a specific biological heat potential and a heat loss flux. Then uses a heat compensation ratio evaluator to compute a transient heat compensation ratio and a minimal heat compensation ratio during the reaction period for evaluating the spontaneity of an ATAT system.

FIG. 1

# EP 1 271 141 A1

**Description**

The present invention relates to a device and method for determining biological heat potential, more specifically to the above-mentioned device and method concerning the autothermal thermophilic aerobic treatment (ATAT) system for wastewater and sludge treatment.

The key problems encountered in the conventional biological wastewater treatment of high-strength wastewaters include high excess sludge yield, low degradation rate, and the low organic loading. An autothermal thermophilic aerobic wastewater treatment (ATAT) system can be used to solve these problems. The ATAT system is normally operated under the aerobic conditions and at temperatures of 45-65°C, differing from those of mesophilic anaerobic treatment at 35°C or thermophilic aerobic treatment at 55°C. According to the vant Hoff-Arrhenius' law, reaction rates easily increase at high temperatures. Moreover, according to bioenergetics, the energy capture efficiency of biomass decreases as the temperature increases, thus increasing the biological reaction temperature will increase the energy part for cell maintenance and simultaneously decrease the part for cell synthesis. Thus, the high temperature of an ATAT can reduces the sludge yield and increase the heat released from maintenance energy.

The current difficulties or misconception in the design of an ATAT system include: requiring costly supplemental heat if the reaction fails to be spontaneous, and limitation of oxygen transfer rate at high degradation rate as dissolved oxygen (DO) concentration decreases at high temperatures. However, in 1990, the United State Environmental Protection Agency (USEPA) reviewed 35 sets of full-scale operating data of autothermal thermophilic aerobic sludge digestion (ATAD) process in Europe and determined that the ATAD is a promising technology for municipal sludge. In Taiwan, ROC, we demonstrated the successful operation of a full-scale ATAT process treating food-processing oily wastewater. Therefore, the ATAT process of high strength wastewater in full-scale was evidenced. However, it requires a reliable device and method to accurately determine the biological heat potential of the wastewater (or sludge) for the successful design and operation of the ATAT process.

If a conventional chemostat is used to evaluate the heat potential of the ATAT process, the problems encountered are: 1. difficulty to evaluate the reaction spontaneity since the system normally results in a large amount of heat loss via poor insulation and aeration; 2. difficulty to overcome the high oxygen transfer rate required by the high degradation of substrate for the ATAT by using a conventional air diffusion system; 3. inaccuracy and time-consuming in the determination of monitoring parameters, such as chemical oxygen demand (COD), biological oxygen demand (BOD), and oxygen uptake rate (OUR) by conventional laboratory methods, particularly for on-line sensing.

The present invention overcomes the difficulties as described above by providing a device and method for determining the biological heat potential and monitoring of heat flux and biological oxygen depletion, particularly for the application of the ATAT process. The invention is featured to provide a pure oxygen supply system, a high-strength mixing device, a surrounding temperature controlled system to minimize heat loss, and a programmable on-line real-time analyzer for transient oxygen uptake and heat supplemental data.

The present invention aims at providing a device to measure an on-line and real-time oxygen uptake data ($O_u$ vs. $t$) and a heat compensation data ($H_c$ vs. $t$) for a biological reaction system.

The present invention also aims at providing a data analysis method to determine the biological heat potential. The method uses a multiple linear regression algorithm with a heat balance model to determine a specific biological heat potential ($h_b$) and a heat loss flux ($J_o$) of an ATAT system.

The device and method used to determine the specific heat potential, as mentioned above, involves an acclimation apparatus for incubating an aerobic microorganism culture, a reactor inoculated by the culture to carry out the desired biological reactions, an external temperature controller to heat and control the ambient air surrounding the reactor at a preset temperature, an oxygen controller to supply and record the oxygen depleted within the reactor with an on-line and real-time output of oxygen uptake data ($O_u$ vs. $t$), an internal heat controller to control and heat the reactor at a preset temperature with an on-line and real-time output of heat compensation data ($H_c$ vs. $t$). Based on the above oxygen uptake data and heat compensation data, the method of this invention uses a specific biological heat potential evaluator to determine the specific biological heat potential ($h_b$) and heat loss flux ($J_o$). The method also uses a heat compensation ratio evaluator to determine a transient heat compensation ratio ($r$) and a minimal heat compensation ratio ($r_{min}$) during the reaction period for evaluating the spontaneity of an ATAT system.

The device mentioned above for determining the biological heat potential mainly consists of six units: a reactor, an oxygen controller, an internal heat controller, an external temperature controller, a mixing controller, and a signal and data controller. The device focuses on the programmable on-line and real-time capability for monitoring a large set of transient oxygen uptake data, heat compensation data, and surrounding temperature data. It also provides a method to use the device as mentioned above to determine the specific biological heat potential ($h_b$) via the heat balance computer algorithm for the ATAT system.

The drawings disclose an illustrative embodiment of the present invention which serves to exemplify the various advantages and objects hereof, and are as follows:

Figure 1 shows the block diagram illustrating the main parts and their relations for the device of the present invention for determining the biological heat potential;

Figure 2 shows the diagram illustrating the acclimation apparatus of the present invention, which provides the necessary aerobic culture for the ATAT test;

Figure 3 shows the diagram illustrating the device of the present invention, used to determine the biological heat potential for the ATAT test;

Figure 4 shows the results of the oxygen uptake data fed on the glucose sample, obtained with the device of the present invention; and

Figure 5 shows the results of heat compensation data fed on the glucose sample as the substrate (energy source), obtained with the device of the present invention.

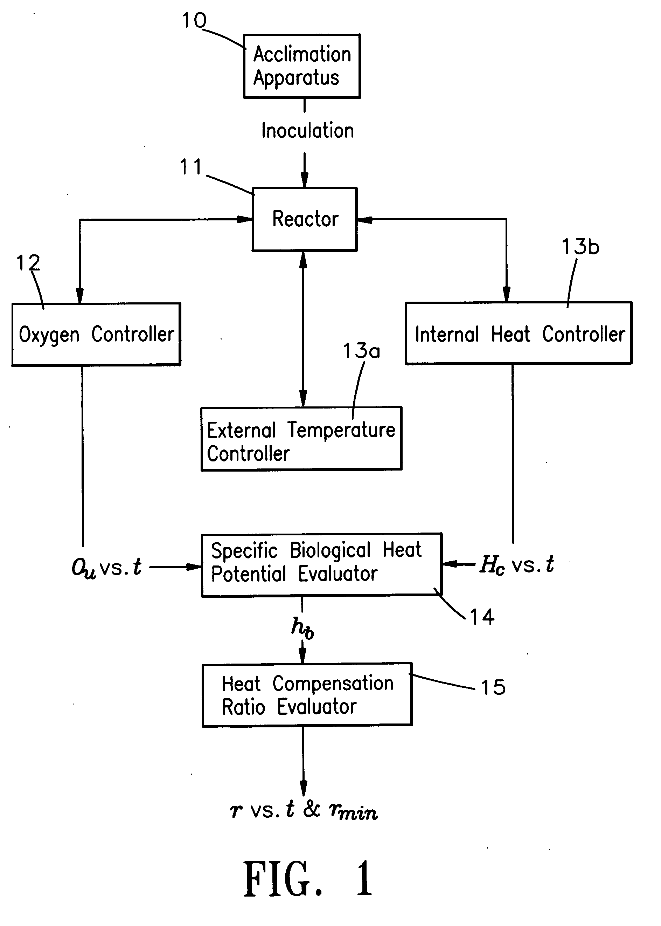

According to Figure 1, it is shown that the present invention provides a device and method for determining the biological heat potential. The device consists of an acclimation apparatus 10 for incubating an aerobic culture which is used as the source culture to a reactor 11 for carrying out the desired biological reactions, an external temperature controller 13a can used to control and heat the ambient air surrounding the reactor 11 at a preset temperature for reducing the heat loss flux during the biological reaction, an oxygen controller 12 to provide and control the oxygen depleted within the reactor 11 with an on-line and real-time output of oxygen uptake data ($O_u$ vs. $t$), an internal heat controller 13b to control and heat the content of the reactor 11 at a preset temperature with an on-line and real-time output of heat compensation data ($H_c$ vs. $t$). Based on the above oxygen uptake data and heat compensation data, the method uses a specific biological heat potential evaluator 14 to compute the specific biological heat potential ($h_b$) and the heat loss flux ($J_o$). The method also uses a heat compensation ratio evaluator 15 to compute a transient heat compensation ratio ($r$), and a minimal heat compensation ratio ($r_{min}$) for evaluating an ATAT system.

### Derivation of the specific biological heat potential algorithm

The specific biological heat potential ($h_b$) of this invention is defined as the released heat of a biological reaction per unit of substrate degraded in terms of biological oxygen demand (BOD), in the unit of kcal/g BODr. It is an important parameter for the design and operation of an ATAT system. The parameter can be included into a heat balance mathematical analysis for evaluating the reaction spontaneity and operating temperature of an ATAT system. For a biological reaction system at steady-state (dT/dt = 0), with the use of the internal heat controller and the external temperature controller, the heat balance analysis around the system can be established by the reaction term, the heat loss term, and the heat compensation term, as follows:

$$0 = \underset{reaction}{h_b \frac{dO_u}{dt}} - \underset{loss}{J_0} + \underset{compensation}{\frac{dH_c}{dt}} \qquad (1)$$

where $h_b$ being the specific biological heat potential in the unit of kcal/g BODr. $O_u$ being the accumulated oxygen uptake in the unit of g BODr, $J_o$ being the heat loss flux in the unit of kcal/min, and $H_c$ being the accumulated heat compensation data in the unit of kcal. Using an initial condition: at $t = 0$, $O_u = 0$ and $H_c = 0$, Equation (1) can be integrated into

$$H_c = J_o t - h_b O_u \qquad (2)$$

Using the device of the present invention, the on-line real-time oxygen uptake data ($O_u$ vs. $t$) and the heat compensation data ($H_c$ vs. $t$) can be measured. These data can be incorporated into Equation (2) and analyzed by a multiple linear regression method based on the least-squared method for determining the specific biological heat potential ($h_b$) and the heat loss flux ($J_o$). During the period of reaction, a heat compensation ratio ($r$) can be defined as follows:

$$r = H_c / J_o t \qquad (3)$$

The magnitude of the r ratio is an indication of reaction spontaneity for the ATAT system. The smaller the r, the larger the extent of reaction spontaneity can be evidenced with the lab-scale ATAT system.

### Acclimation apparatus

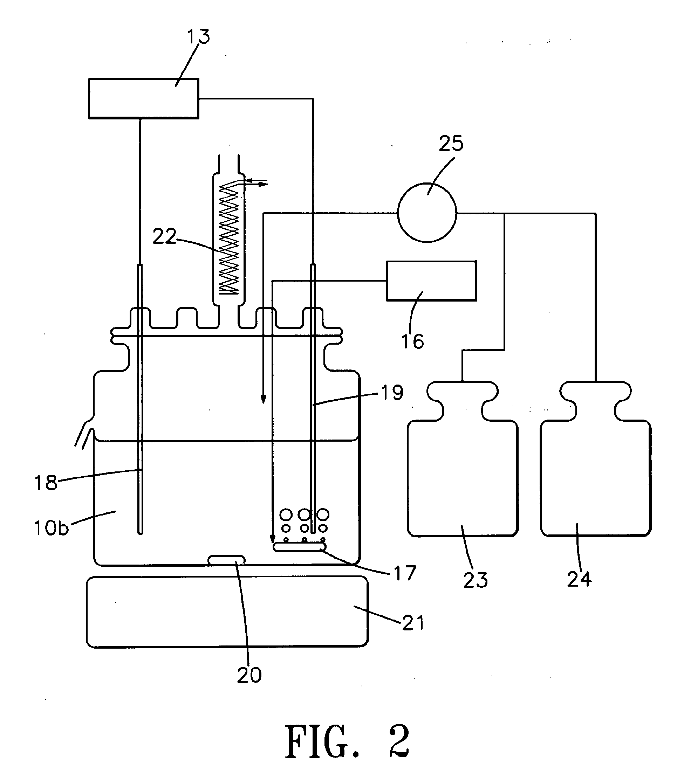

The present invention provides an acclimation apparatus for incubating an aerobic culture, as shown in Figure 2. It consists of a 11 liter acclimation reactor 10b with a liquid volume of 10 liter, an air pump 16 and a diffuser 17 to maintain the dissolved oxygen (DO) concentration at 1 to 2 mg/L, a temperature probe 18 and a heater 19, incorporated with a proportional integrated derivative (PID) temperature controller 13b to maintain the acclimation reactor 10b at 55°C with a precision of 0.1°C, a heavy-duty magnate 20 driven by a magnetic stirrer 21 to maintain a homogeneous content within the acclimation reactor 10b, a reflux condenser 22 connected to the top the acclimation reactor 10b to prevent the loss of liquid solution via vaporization, a 4 liter substrate vessel 23 and a 4 liter nutrient vessel 24 to avoid the undesired substrate spoiling, and a peristaltic pump 25 to feed the acclimation reactor 10b operated at a preset flow rate and sludge retention time (SRT) for a treatment efficiency.

### Example:

### Acclimation operation

Firstly, a 10 liter sludge is transferred from the aeration tank of a full-scale ATAT plant to the reactor of the acclimation apparatus. The reactor is fed on a glucose sample at a loading of 10 g/L COD and an SRT of 10 days. Effluent samples are periodically taken from the reactor for the analyses of COD, pH, and MLSS. Other performance characteristics are also observed for sludge settlability, culture color, forming, and microscope examination to determine the acclimation status.

The present invention allows for maintaining the normal acclimation operation to provide a stable source of seed culture required for the ATAT tests.

### ATAT test

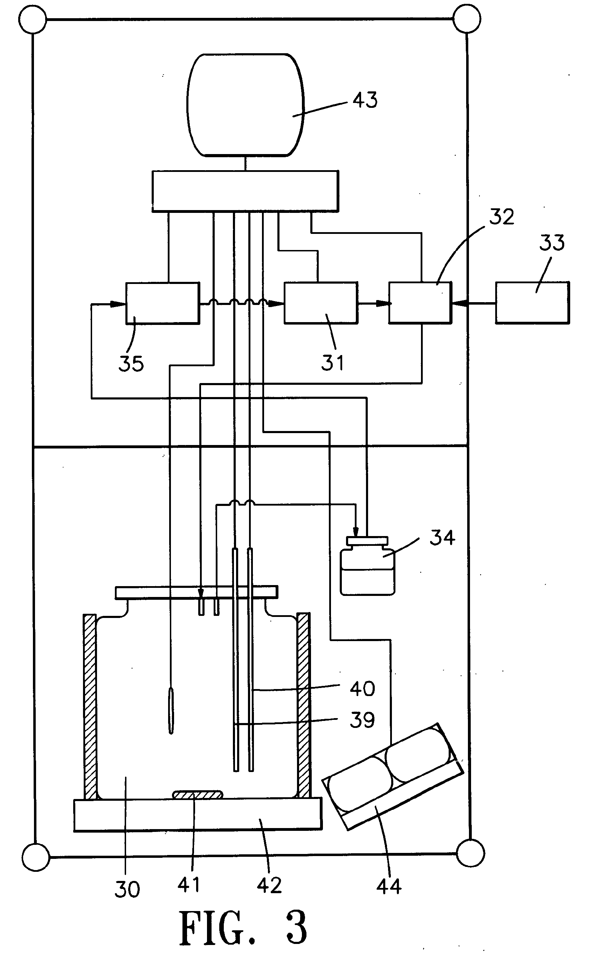

The ATAT test, as shown in Figure 3, consists of a reactor, an oxygen controller, an internal heat controller, an external temperature controller, a mixing controller, and a signal and data controller capable of the on-line and real-time data acquisition of an oxygen uptake data and a heat compensation data. It can be used for an ATAT system and other biological reaction studies to obtain the specific biological heat potential based on the heat balance analysis.

As shown in Figure 3, the oxygen controller provides pure oxygen to maintain the oxygen content of the headspace of the reactor 30 at a preset value. The oxygen supply is regulated by a computer program with an on/off control mechanism 32. The oxygen source 33 will be turned on if oxygen level determined by the oxygen probe 31 is below the preset value and is turned off if above the preset value. Providing the oxygen is supplied at a constant rate, the on-time as recorded by the on-line and real-time computer program can be used to calculate the accumulated oxygen uptake data ($O_u$ vs. $t$). The carbon dioxide ($CO_2$) stripper 34 is used to absorb the released $CO_2$ resulting from the aerobic reaction by strong basic solution, such as potassium hydroxide (KOH). The internal heat controller uses an on/off control system, referring to the internal temperature probe 39 and the internal heater 40 to allow for operation of the reactor 30 at a constant temperature. Providing the heating power of the internal heater 40 is kept at constant, the on-line and real-time computer program can record the on-time of the internal heater 40 to calculate the heat compensation data ($H_c$ vs. $t$). The magnate 41 is driven by the magnetic stirrer 42 for high-strength mixing within the reactor 30 at a controllable speed to avoid spin-off. The signal and data controller, comprising a signal interface assembly 38 and a signal and data processor 43, consists of an on-line and real-time computer program, which can be used to monitor, control, and exhibit dynamic plots of the mixing speed, the reactor and surrounding air temperatures, the oxygen uptake data, and the heat compensation data. The external temperature controller uses the temperature probe 37 connected to the external heater 44 to maintain at an appropriate constant surrounding temperature to reduce heat loss from the reactor 30. The ambient air surrounding the reactor 30 is circulated with a fan to maintain a homogeneous ambient temperature.

When operating the ATAT test, the seed culture is first transferred into the reactor 30 and then operated in a semi-continuous mode. The substrate (such as glucose) sample is daily fed after daily wasting of the excess sludge from the reactor 30 to control the ATAT test at a preset SRT. After reaching a steady state, a repeatable pattern of oxygen uptake data can be observed and the ATAT test is completed.

**Results and Discussion**

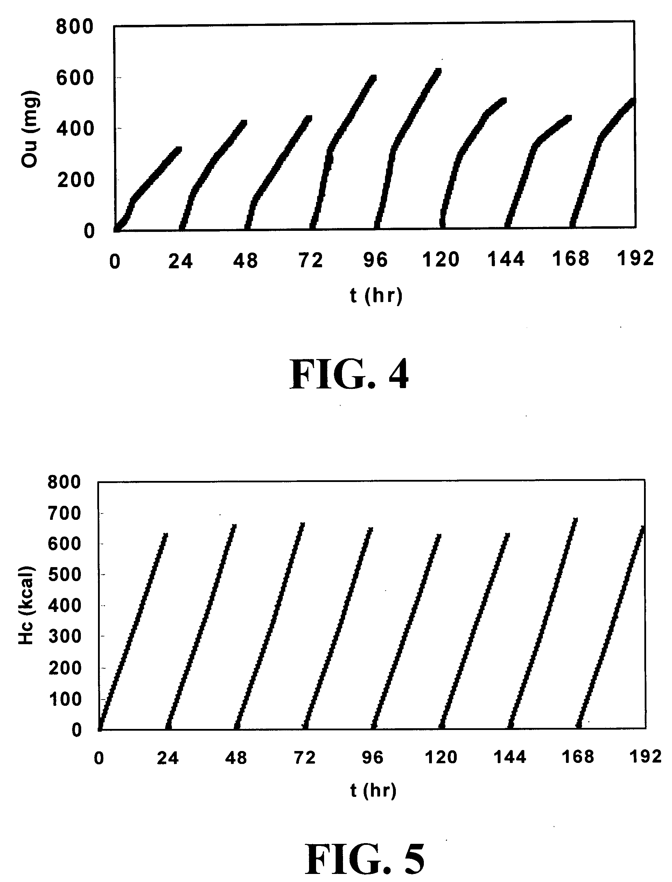

Figures 4 and 5 show the $O_u$ and $H_c$ curves. It can be found that the $O_u$ curve varied from day to day, and the $H_c$ curve was more repeatable during one day of testing period. The $O_u$ data and $H_c$ data were analyzed with Equation (3) with a multiple linear regressed method to estimate the specific biological heat potential ($h_b$) and the heat loss flux ($J_o$) for comparison. The regression results indicate that the $h_b$ normally ranges from 3.28-4.53 kcal/g BODr, with exception from the 3rd to 6th days. The $R^2$ values of 0.92-0.95 for all the 8 testing days indicate good linearity, even for 3rd to 6th days. The reason for the wide variation of $h_b$ from the 3rd to 6th days remains to be further investigated. It was speculated that it might be due to the dynamic nature of the thermophilic microbial system associated with incomplete acclimation under the testing conditions. It was not likely due to the mechanical problems of the ATAT system since $h_b$ was automatically brought back to the base line values of the 1st to 3rd days at the 7th and 8th days.

The regression result also indicates that the heat loss flux ($J_o$) varies in a fairly narrow range of 0.43-0.55 kcal/min. The heat loss flux reduces about 10% when the system experienced lower biological heat on the 5th to 6th days.

Using the average $h_b$ value (4.28 kcal/g BODr) and the average heat loss flux ($J_o$) value (0.50 kcal/min), the heat compensation ratio can be determined to be with a minimum of 89.2% at 12 hours during the 24 hours of testing period.

The present invention provides a device for determining the biological heat potential by measuring the on-line real-time oxygen uptake data ($O_u$ vs. $t$) and heat compensation data ($H_c$ vs. $t$) from an ATAT test. The present invention also provides a data processing method to calculate the biological heat potential. The method uses a multiple linear regression algorithm to solve a governing function based on a heat balance model to determine the specific biological heat potential ($h_b$) and the heat loss flux ($J_o$) of the ATAT test. The results show that the present invention is a useful tool for an ATAT study. As the system is fed on 10-g COD/L glucose sample at an SRT of 10 days, the $h_b$ is determined to be 4.28 kcal/g BODr. Moreover, the heat loss flux ($J_o$) of the ATAT system is determined to be 0.50 kcal/min and the minimal heat compensation ratio ($r_{min}$) is 89.2%.

Many changes and modifications in the above described embodiment of the invention can, of course, be carried out without departing from the scope thereof. Accordingly, to promote the progress in science and the useful arts, the invention is disclosed and is intended to be limited only by the scope of the appended claims.

**Claims**

1. A method for determining biological heat potential of biological reaction systems, particularly the ATAT system, which comprising the following steps:

    using an acclimation apparatus to incubate an aerobic culture and transferring the culture into a reactor for the biological reaction test;
    controlling, heating, and recording an ambient air surrounding the reactor at a first preset temperature;
    controlling and supplying a pure oxygen to maintain the oxygen level at a preset value in a headspace of the reactor, and recording an on-line and real-time oxygen uptake data ($O_u$ vs. $t$);
    controlling and heating a content of the reactor at a second preset temperature, and recording an on-line and real-time heat compensation data ($H_c$ vs. $t$);
    using the oxygen uptake data and the heat compensation data to compute a specific biological heat potential ($h_b$) and a heat loss flux ($J_o$); and
    using the calculated specific biological heat potential ($h_b$) and the heat loss flux ($J_o$) to compute a transient heat compensation ratio ($r$) and a minimal heat compensation ratio ($r_{min}$) during a reaction period.

2. A method for determining biological heat potential as recited in claim 1, wherein the on-line and real-time oxygen uptake data is obtained with an oxygen controller, which comprising means for controlling and providing the oxygen depleted in the reactor.

3. A method for determining biological heat potential as recited in claim 1, wherein the on-line and real-time heat compensation data is obtained with a heat compensation controller, which comprising means for controlling and heating the content of the reactor at the second preset temperature.

4. A method for determining biological heat potential as recited in claim 1, wherein the on-line and real-time oxygen uptake data ($O_u$ vs. $t$) and the heat compensation data ($H_c$ vs. $t$) are analyzed by a heat balance equation, considering a reaction term, a heat loss term, and a heat compensation term:

$$0 = \underset{reaction}{h_b \frac{dO_u}{dt}} - \underset{loss}{J_0} + \underset{compensation}{\frac{dH_c}{dt}} \qquad (1)$$

where $h_b$ being the specific biological heat potential in the unit of kcal/g BODr, $O_u$ being the accumulated oxygen uptake data in the unit of g, $J_o$ being the heat loss flux in the unit of kcal/min, and $H_c$ being the accumulated compensated heat in the unit of kcal; and

integrating equation (1) with the initial conditions of $t = 0$, $O_u = 0$, and $H_c = 0$ to obtain

$$H_c = J_o t - h_b O_u \qquad (2).$$

5. A method for determining biological heat potential as recited in claim 4, wherein the specific biological heat potential ($h_b$) and the heat loss flux ($J_o$) are used to calculate a heat compensation ratio ($r$):

$$r = H_c / J_o t \qquad (3).$$

6. A device for determining biological heat potential comprising:

an acclimation apparatus to incubate an aerobic seed culture;
a reactor receiving the seed culture from the acclimation apparatus to carry out a biological reaction;
an external temperature controller to control and heat an ambient air surrounding the reactor at a first preset temperature;
an oxygen controller to provide and record an oxygen depleted within the reactor with an on-line and real-time output of oxygen uptake data ($O_u$ vs. $t$);
an internal heat controller to control and heat the reactor at a second preset temperature with an on-line and real-time output of heat compensation data ($H_c$ vs. $t$);
a specific biological heat potential evaluator using the on-line and real-time data of oxygen uptake and heat compensation to determine a specific biological heat potential ($h_b$) and a heat loss flux ($J_o$);
a heat compensation ratio evaluator using the specific biological heat potential ($h_b$) and the heat loss flux ($J_o$) to determine a heat compensation ratio ($r$) and a minimal heat compensation ratio ($r_{min}$) during a reaction period.

7. A device for determining biological heat potential as recited in claim 6, wherein the acclimation apparatus comprises an air pump for aeration via a fine-bubble diffuser on a bottom portion the reactor, a temperature probe connected to a heater and a proportional integrated derivative (PID) temperature controller to maintain the reactor at a constant temperature, a heavy-duty magnate on the bottom portion of the reactor driven by a magnetic stirrer for homogenous mixing, and two glass bottles for storing a substrate solution and a nutrient solution being fed simultaneously by a peristaltic pump at a preset flow rate to maintain at a preset sludge retention time (SRT) and substrate concentration (S) for the reactor.

8. A device for determining biological heat potential as recited in claim 7, wherein a reflux condenser is connected to a top portion of the acclimation reactor to prevent a loss of liquid solution via evaporation.

9. A device for determining biological heat potential as recited in claim 6, wherein comprising means for evaluating the spontaneity of an autothermal thermophilic aerobic treatment system.

10. A device for determining biological heat potential comprising a reactor, an oxygen controller, an internal heat controller, an external temperature controller, a mixing controller, and a signal and data controller with the following characteristics:

comprising means for programmable on-line and real-time monitor and control of a transient oxygen uptake data and a heat compensation data, and using a heat balance analysis to process a specific biological heat potential algorithm for biological reaction thermodynamics and autothermal thermophilic aerobic treatment feasibility study.

**11.** A device for determining biological heat potential as recited in claim 10, wherein the oxygen controller, according to the estimate by an oxygen probe, uses a computer program with an on/off control mechanism connecting to an oxygen source to provide a preset flowrate of oxygen per on-time, which can be recorded and calculated to be the oxygen uptake data, and a carbon dioxide formed during oxidation is absorbed by a strong basic solution within a $CO_2$ stripper.

**12.** A device for determining biological heat potential as recited in claim 10, wherein the heat compensation temperature controller, according to the estimate of a temperature probe, uses a computer program with an on/off control mechanism connecting a heater with a fixed power to maintain the temperature of the reactor at a preset temperature, which can be recorded and calculated to be the heat compensation data.

**13.** A device for determining biological heat potential as recited in claim 10, wherein the mixing controller comprises a magnate on a bottom portion of the reactor driven by a magnetic stirrer for high-strength mixing.

**14.** A device for determining biological heat potential as recited in claim 11, wherein the signal and data controller comprises a signal and data processor and a signal interface assembly and an on-line and real-time computer program, which contains means for monitoring, controlling, and showing dynamic plots of a mixing speed of the magnetic stirrer, an internal and external temperatures of the reactor, the oxygen uptake data, and the heat compensation data.

**15.** A device for determining biological heat potential as recited in claim 10, wherein the external temperature controller comprising an external temperature probe on an external portion of the reactor and an external heater can be incorporated to a computer program for maintaining the external portion of the reactor at a high temperature for reducing the heat loss.

10

Acclimation
Apparatus

Inoculation

11

Reactor

12

Oxygen Controller

13a

External Temperature
Controller

13b

Internal Heat Controller

$O_u$ vs. $t$

Specific Biological Heat
Potential Evaluator

$H_c$ vs. $t$

14

$h_b$

Heat Compensation
Ratio Evaluator

15

$r$ vs. $t$ & $r_{min}$

# FIG. 1

FIG. 2

FIG. 3

FIG. 4

FIG. 5

## EUROPEAN SEARCH REPORT

**European Patent Office**

**Application Number**

EP 01 11 5125

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y<br>A | WO 01 34527 A (STOVER ENOS L)<br>17 May 2001 (2001-05-17)<br>* the whole document * | 1-4,<br>10-12,14<br>6 | G01N33/18<br>C12M1/00<br>C02F3/00<br>C02F3/02 |
| Y | LAPARA T M ET AL: "Thermophilic aerobic biological wastewater treatment"<br>WATER RESEARCH, ELSEVIER SCIENCE PUBLISHERS, AMSTERDAM, NL,<br>vol. 33, no. 4, March 1999 (1999-03),<br>pages 895-908, XP004155388<br>ISSN: 0043-1354<br>* the whole document * | 1-4 | |
| Y | WO 95 03254 A (BIOCHEM TECHNOLOGY INC)<br>2 February 1995 (1995-02-02)<br>* page 1 - page 14 * | 10-12,14 | |
| A | US 6 133 021 A (GU MAN BOCK ET AL)<br>17 October 2000 (2000-10-17)<br>* column 1, line 55 - column 3, line 67 * | 7 | |
| A | PATENT ABSTRACTS OF JAPAN<br>vol. 017, no. 113 (C-1033),<br>9 March 1993 (1993-03-09)<br>& JP 04 299973 A (HITACHI LTD;OTHERS: 01),<br>23 October 1992 (1992-10-23)<br>* abstract * | 7 | TECHNICAL FIELDS SEARCHED (Int.Cl.7)<br><br>G01N<br>C12M<br>C02F |
| A | US 4 915 840 A (ROZICH ALAN F)<br>10 April 1990 (1990-04-10)<br>* page 1, line 12 - page 7, line 44; figure 1 * | 1-15 | |
| A | US 6 203 701 B1 (PRESSLEY RICHARD L ET AL) 20 March 2001 (2001-03-20)<br>* column 1, line 33 - column 4, line 54 * | 1-15 | |

The present search report has been drawn up for all claims

| Place of search | Date of completion of the search | Examiner |
|---|---|---|
| THE HAGUE | 11 December 2001 | Joyce, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 1 271 141 A1**

**ANNEX TO THE EUROPEAN SEARCH REPORT
ON EUROPEAN PATENT APPLICATION NO.**     EP 01 11 5125

This annex lists the patent family members relating to the patent documents cited in the above-mentioned European search report.
The members are as contained in the European Patent Office EDP file on
The European Patent Office is in no way liable for these particulars which are merely given for the purpose of information.

11-12-2001

| Patent document cited in search report | | Publication date | Patent family member(s) | | Publication date |
|---|---|---|---|---|---|
| WO 0134527 | A | 17-05-2001 | WO | 0134527 A1 | 17-05-2001 |
| | | | AU | 1715100 A | 06-06-2001 |
| WO 9503254 | A | 02-02-1995 | US | 5401412 A | 28-03-1995 |
| | | | US | 5466604 A | 14-11-1995 |
| | | | US | 5552319 A | 03-09-1996 |
| | | | AU | 679772 B2 | 10-07-1997 |
| | | | AU | 7364494 A | 20-02-1995 |
| | | | BR | 9407198 A | 17-09-1996 |
| | | | CA | 2167514 A1 | 02-02-1995 |
| | | | CN | 1127500 A | 24-07-1996 |
| | | | EP | 0710218 A1 | 08-05-1996 |
| | | | FI | 960261 A | 19-01-1996 |
| | | | HU | 77644 A2 | 29-06-1998 |
| | | | JP | 9500277 T | 14-01-1997 |
| | | | NO | 960212 A | 17-01-1996 |
| | | | NZ | 269942 A | 24-11-1997 |
| | | | RU | 2134661 C1 | 20-08-1999 |
| | | | TW | 432017 B | 01-05-2001 |
| | | | WO | 9503254 A1 | 02-02-1995 |
| US 6133021 | A | 17-10-2000 | JP | 3141008 B2 | 05-03-2001 |
| | | | JP | 11316219 A | 16-11-1999 |
| JP 04299973 | A | 23-10-1992 | NONE | | |
| US 4915840 | A | 10-04-1990 | NONE | | |
| US 6203701 | B1 | 20-03-2001 | US | 5948261 A | 07-09-1999 |
| | | | US | 6168717 B1 | 02-01-2001 |
| | | | AU | 6068400 A | 22-01-2001 |
| | | | WO | 0102308 A1 | 11-01-2001 |
| | | | AU | 2495299 A | 23-08-1999 |
| | | | WO | 9940036 A1 | 12-08-1999 |
| | | | US | 2001001454 A1 | 24-05-2001 |

EPO FORM P0459

For more details about this annex : see Official Journal of the European Patent Office, No. 12/82

13